(19) Europäisches Patentamt European Patent Office Office européen des brevets

(11) **EP 4 454 565 B1**

(12) **EUROPEAN PATENT SPECIFICATION**

(45) Date of publication and mention
of the grant of the patent:
**30.04.2025   Bulletin 2025/18**

(21) Application number: **23180202.6**

(22) Date of filing: **20.06.2023**

(51) International Patent Classification (IPC):
**A61B 5/352** $^{(2021.01)}$     **A61B 5/366** $^{(2021.01)}$
**A61B 5/00** $^{(2006.01)}$

(52) Cooperative Patent Classification (CPC):
**A61B 5/352; A61B 5/366; A61B 5/725; A61B 5/308**

(54) **DEVICE FOR AUTOMATIC QRS COMPLEX DETECTION IN ELECTROCARDIOGRAM SIGNAL**

VORRICHTUNG ZUR AUTOMATISCHEN QRS-KOMPLEX-DETEKTION IN EINEM
ELEKTROKARDIOGRAMMSIGNAL

DISPOSITIF DE DÉTECTION AUTOMATIQUE DE COMPLEXE QRS DANS UN SIGNAL
D'ÉLECTROCARDIOGRAMME

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **28.04.2023   PL 44466923**

(43) Date of publication of application:
**30.10.2024   Bulletin 2024/44**

(73) Proprietor: **Akademia Gorniczo-Hutnicza im.
Stanislawa
Staszica w Krakowie
30-059 Krakow (PL)**

(72) Inventors:
• **Szaflarski, Andrzej**
  **Szaflary 34-424 (PL)**
• **Reklewski, Wojciech**
  **02-493 Warszawa (PL)**
• **Heryan, Katarzyna**
  **30-109 Kraków (PL)**
• **Kisiel-Dorohinicki, Marek**
  **Kraków 31-463 (PL)**
• **Miskowicz, Marek**
  **31-621 Kraków (PL)**

(74) Representative: **Wlasienko, Jozef et al
Polservice
Kancelaria Rzeczników
Patentowych Sp. z o.o.
Ul. Bluszczanska 73
00-712 Warszawa (PL)**

(56) References cited:
**EP-A1- 3 970 616**

• **GUTIERREZ-RIVAS RAQUEL ET AL: "Novel Real-Time Low-Complexity QRS Complex Detector Based on Adaptive Thresholding", IEEE SENSORS JOURNAL, IEEE, USA, vol. 15, no. 10, 1 October 2015 (2015-10-01), pages 6036 - 6043, XP011666999, ISSN: 1530-437X, [retrieved on 20150818], DOI: 10.1109/JSEN.2015.2450773**
• **JAIN SHWETA ET AL: "QRS detection using adaptive filters: A comparative study", ISA TRANSACTIONS, INSTRUMENT SOCIETY OF AMERICA. PITTSBURGH, US, vol. 66, 10 October 2016 (2016-10-10), pages 362 - 375, XP029903602, ISSN: 0019-0578, DOI: 10.1016/ J.ISATRA.2016.09.023**
• **XIAO RUPING ET AL: "Ultra-low power QRS detection using adaptive thresholding based on forward search interval technique", 2017 INTERNATIONAL CONFERENCE ON ELECTRON DEVICES AND SOLID-STATE CIRCUITS (EDSSC), IEEE, 18 October 2017 (2017-10-18), pages 1 - 2, XP033274171, DOI: 10.1109/ EDSSC.2017.8126486**

**Description**

The subject of the invention is a device for QRS complex detection in an electrocardiogram signal ECG applicable in biomedical diagnostics.

State of the art

Electrocardiography is one of the most commonly performed diagnostic procedures that allows the analysis and assessment of the heart's electrical activity. Reading the potential difference between two electrodes placed on the body of a patient, mostly on the chest, allows plotting the so-called electrocardiographic curve (ECG curve) representing the course of the mentioned potential difference over time. Evaluation of the ECG curve requires analysis to be made by a doctor or a specialist in order to detect possible irregularities of the heart activity of the examined person. In order to support the interpretation and evaluation of the ECG curve, many devices and algorithms have been developed that allow to at least partially automate the ECG curve analysis, increase its reliability and accelerate the diagnosis.

The ECG curve is cyclical due to the repetitive activity of the heart. In each single cycle of the ECG curve, there are characteristic waveform features corresponding to cardiac activity, which most often include the P-wave, the QRS complex and the T-wave. The P-wave corresponds to depolarization of the atrial muscle, the QRS complex corresponds to depolarization of the ventricular muscle, and the T-wave corresponds to ventricular repolarization. The description of the heart's activity and the interpretation of the ECG curve is the subject of numerous studies available in the art.

In order to enable an automated analysis of the ECG curve, it is essential to reliably detect the characteristic features of the ECG curve, in particular the above-mentioned QRS complex.

From the publication Gutiérrez-Rivas, Raquel, et al. "Novel real-time low-complexity QRS complex detector based on adaptive thresholding." IEEE Sensors Journal, vol. 15, no. 10, s. 6036-6043, 2015 a QRS complex detector based on adaptive thresholding is known.

From the application P.435396 a device for detecting the QRS complex of an electrocardiogram signal is known, that has modules ABS_DIFF_SHORT_MODULE and ABS_DIFF_LONG_MODULE, the inputs of which are connected to the ECG_MODULE measuring module output, and the ABS_DIFF_SHORT_MODULE module output is connected to one of the COMP comparator inputs, and the output of the ABS_DIFF_LONG_MODULE module is connected to the input of the PEAK_DETECTOR detection module. The COMP comparator output is connected to the PULSE GENERATOR pulse generator input. The output of the PULSE GENERATOR pulse generator is connected to the gating input of the PEAK_DETECTOR detection module and to the R_AMPLITUDE_MEMORY memory recording module input and to the R_TIMESTAMP_MEMORY memory recording module input. The output of the PEAK_DETECTOR detection module is also connected to the R_AMPLITUDE_MEMORY and R_TIMESTAMP_MEMORY memory module inputs, and the R_AMPLITUDE_MEMORY memory module output is connected to the TH_MODULE module input. The TH_MODULE module output is also connected to one of the COMP comparator inputs.

From the application P.435394 a method of detecting QRS complex of electrocardiogram signal is also known.

The object of the present invention is to provide an improved device for automatic QRS complex detection in an electrocardiogram signal with increased reliability of R-wave detection. This object is solved by the device according to claim 1.

According to the present invention, a device for automatic QRS complex detection in an electrocardiogram signal having connection with a measurement module ECG MODULE of the ECG signal is provided, wherein the device comprises:

- a module BUF for buffering data supplied to its input connected to an output of the module ECG MODULE,

- a module PREPROCESSING MODULE, whose first input is connected to an output of the module BUF, generating at its output a signal Y_PREPROCESSED obtained by generating a signal $Y0$ being the difference between a value $Y$ of the considered sample of the ECG signal and a sample value of the ECG signal previously stored in the buffer BUF, which has been received a predetermined number $Nd$ of samples earlier, and determining a signal $Y1$ being the sum of the considered sample of the signal $Y0$ and a value of a predetermined number N-1 of samples of the signal $Y0$ immediately preceding the considered sample of the signal $Y0,$ and dividing this sum by a number N and squaring such obtained number.

- a module TH MODULE for setting an initial value and determining a current threshold value TH_PREPROCESSED;

- a comparator COMPARATOR, whose first input is connected to the output of the module PREPROCESSING MODULE, and whose second input is connected to an output of the TH MODULE;

- a module SEARCHING WINDOW PULSE GENERATOR generating a time window SEARCHING_WINDOW for searching for the R-wave in the QRS complex of the ECG signal, whose first input is connected to an output of the comparator COMPARATOR, and whose output is connected to a second input of the module PREPROCESSING MODULE,

- a module PEAK DETECTOR for detecting a maximum value of the signal Y_PREPROCESSED, whose first input is connected to the output of the module PREPROCESSING MODULE and whose second input is connected to the output of the module SEARCHING WINDOW PULSE GENERATOR,

- a module PEAK AMPLITUDE MEMORY for storing a maximum value of the signal Y_PREPROCESSED, whose input is connected to a first output of the module PEAK DETECTOR and whose output is connected to an input of the module TH MODULE,

- memory modules AMPLITUDE MEMORY and TIMESTAMP MEMORY for storing, respectively, an amplitude value and occurrence time of a parameter corresponding to the detected R-wave, as well as a memory module OUTPUT MEMORY for storing values from the memories AMPLITUDE MEMORY and TIMESTAMP MEMORY, whose outputs are connected to inputs of the memory module OUTPUT MEMORY.

[a] the device according to the invention is characterized in that furthermore comprises:

- a module PULSE EXTENSION for detecting that the maximum value of the signal Y_PREPROCESSED reduced by the current value of the signal Y_PREPROCESSED reaches a predetermined threshold MIN_DIFF, whose first input is connected to the output of the module PREPROCESSING MODULE, whose second input is connected to the output of the module PEAK AMPLITUDE MEMORY and whose output is connected to a second input of the module SEARCHING WINDOW PULSE GENERATOR,

- a module REFRACTORY WINDOW PULSE GENERATOR for measuring a time window REFRACTORY_WINDOW, the end of which occurs after time REFRACTORY_LEN since a time instant of detecting the last R-wave, whose input is connected to a second output of the module PEAK DETECTOR and whose output is connected to a third input of the module PREPROCESSING MODULE,

- a module EXTREMA MODULE for searching for local extremes of the ECG signal, whose first input is connected to the output of the module BUF, whose second input is connected to the output of the module REFRACTORY WINDOW PULSE GENERATOR, and whose third input is connected to the second output of the module SEARCHING WINDOW PULSE GENERATOR,

- a module FACTOR MODULE for determining a value of a parameter FACTOR, which specifies how distinct local extreme the considered sample of the ECG signal is against the neighbouring samples of the ECG signal, whose input is connected to an output of the module EXTREMA MODULE,

- a module TH FACTOR MODULE for determining and updating a threshold TH_FACTOR, whose input is connected to the output of the module AMPLITUDE MEMORY,

- a memory module FACTOR MEMORY for storing a list THRESHOLD_WINDOW_LIST comprising values of the parameter FACTOR greater than the set threshold TH_FACTOR and time instants of receiving associated samples of the ECG signal, for which the parameter FACTOR has been determined, whose first input is connected to an output of the module FACTOR MODULE, whose second input is connected to the output of the module REFRACTORY WINDOW PULSE GENERATOR, whose third input is connected to the output of the module SEARCHING WINDOW PULSE GENERATOR, and whose fourth input is connected to an output of the module TH FACTOR MODULE,

- a module FACTOR SELECTOR for selecting the greatest one among the values stored in the list SEARCHING_WINDOW_LIST, whose first input is connected to an output of the module FACTOR MEMORY, whose second input is connected to the output of the module SEARCHING WINDOW PULSE GENERATOR, and whose two outputs are connected, respectively, to the module TIMESTAMP MEMORY and to the module AMPLITUDE MEMORY.

The device according to the invention provides increased reliability of detecting R-waves even in the presence of cardiac anomalies and in the presence of interference or distortion of the ECG signal. The structure of the device furthermore makes it possible to keep the computational complexity low, whereby the device can be implemented as a portable device.

The subject of the invention in an embodiment is illustrated in the drawing, in which:

Fig. 1 is a simplified diagram of an electrocardiogram signal waveform with marked P- and T-waves and QRS complex;
Fig. 2 is a block diagram of a device for automatic QRS complex detection in an electrocardiogram signal according to the present invention.

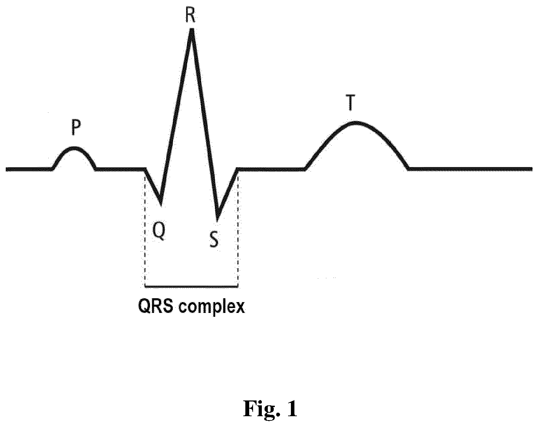

Fig. 1 is a simplified diagram of an electrocardiogram signal waveform (ECG) with marked P- and T- waves and QRS complex. Such electrocardiogram signal (ECG) is received by means of a measuring module through electrodes placed on the body of a patient. The ECG signal is characterized by a sequence of positive and negative deviations (so-called waves) from the isoelectric line, which corresponds to periods of time in which no cardiac excitation is found. The group of the largest waves includes a negative deflection (Q-wave), a positive deflection (R-wave) and again a negative deflection (S-wave) and is referred to as the so-called QRS complex. The greatest amplitude in the QRS complex usually has the R-wave, so that the detection of the QRS complex often comes down to detection of the R-wave. The statistics of time intervals between R-waves and of amplitudes of the R-waves is an important diagnostic information used in medicine, among others, to identify the heart activity.

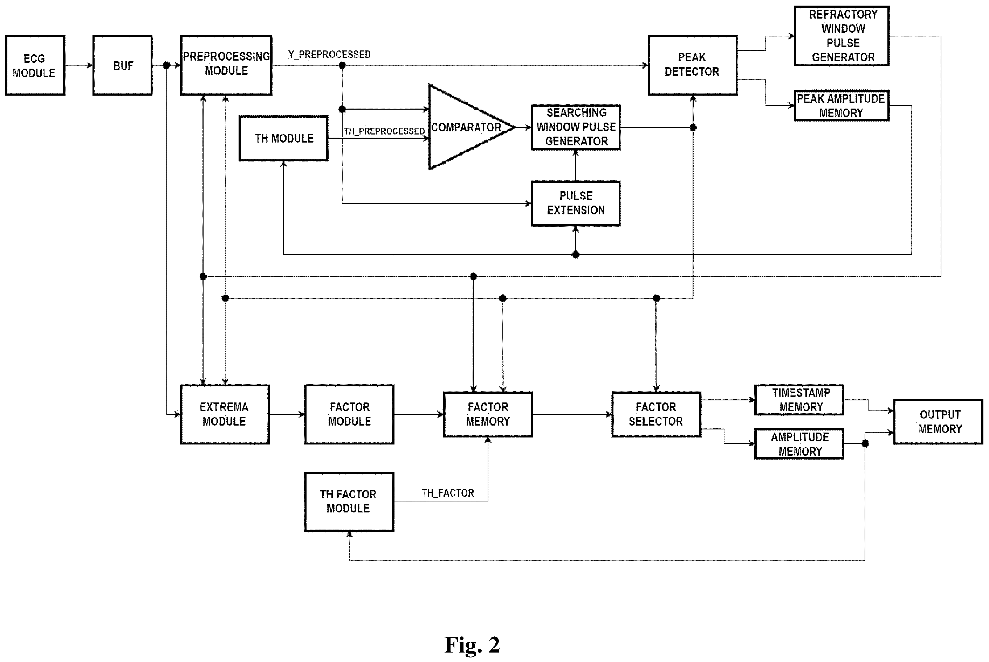

Fig. 2 is a block diagram of a device for automatic QRS complex detection in an electrocardiogram signal according to the present invention. As shown in the figure 2, [a] the device according to the present invention comprises a number of mutually connected modules, which are assigned specific functions necessary for R-wave detection of QRS complex. According to the presented block diagram, an output of a module ECG MODULE is connected to an input of a buffer module BUF, whose output is connected to an input of a module PREPROCESSING MODULE. Outputs of the module PREPROCESSING MODULE and of a module TH MODULE are connected to inputs of a comparator COMPARATOR, whose output is connected to a module SEARCHING WINDOW PULSE GENERATOR. Inputs of a module PEAK DETECTOR are connected to outputs of the modules PREPROCESSING MODULE and SEARCHING WINDOW PULSE GENERATOR, respectively, whereas its outputs are connected to inputs of modules REFRACTORY WINDOW PULSE GENERATOR and PEAK AMPLITUDE MEMORY, respectively. Inputs of a module PULSE EXTENSION are connected to outputs of the modules PREPROCESSING MODULE and PEAK AMPLITUDE MEMORY, respectively, whereas its output is connected to an input of the module SEARCHING WINDOW PULSE GENERATOR. Inputs of a module EXTREMA MODULE are connected to inputs of the modules BUF, REFRACTORY WINDOW PULSE GENERATOR, SEARCHING WINDOW PULSE GENERATOR, respectively, whereas its output is connected to an input of a module FACTOR MODULE. Particular inputs of a module FACTOR MEMORY are connected to outputs of modules REFRACTORY WINDOW PULSE GENERATOR, SEARCHING WINDOW PULSE GENERATOR and TH FACTOR MODULE, respectively. A module FACTOR SELECTOR has two inputs connected to the modules FACTOR MEMORY and SEARCHING WINDOW PULSE GENERATOR, respectively, and two outputs connected to modules TIMESTAMP MEMORY and AMPLITUDE MEMORY, whose outputs are connected to inputs of a module OUTPUT MEMORY. The output of the module AMPLITUDE MEMORY is also connected to an input of the module TH FACTOR MODULE. Functions of the individual modules and the operation principle of the device according to the invention are explained in the following detailed description of an embodiment.

The measuring module ECG MODULE provides an electrocardiogram signal (ECG) in the form of signal samples, which are delivered at a given frequency determined by a signal CLK, which frequency in the embodiment is 360Hz. By means of the module TH MODULE an initial value of a threshold TH_PREPROCESSED is set, equal to a maximum value of a signal Y_ PREPROCESSED detected within first 3 seconds of the ECG signal obtained by means of the measuring module ECG MODULE, and an initial value of a threshold TH_FACTOR is set by means of the module TH FACTOR MODULE, equal to a parameter MIN_TH_FACTOR = 1.22 [mV]. The full range of possible changes of the analogue ECG signal is +5mV. The resolution of the A/D converter in this embodiment is $2^{11}=2048$, and therefore 1.22 mV corresponds to 244 "steps" of the A/D converter. The resolution of the A/D converter is 5μV. Next, a signal Y_PREPROCESSED generated at the output of the module PREPROCESSING MODULE is monitored by means of a comparator COMPARATOR, which signal is obtained by:

generating by means of the module PREPROCESSING MODULE a signal *Y0* being the difference between a value *Y* of the considered sample of the ECG signal and a sample value of the ECG signal previously stored in the buffer BUF, which has been received a predetermined number *Nd* = 7 samples earlier (for signal CLK=360Hz, which corresponds to time 19.4445 ms),

and determining by means of the module PREPROCESSING MODULE a signal *Y1,* which is the sum of the value of the signal *Y0* of the considered sample of the ECG signal and a value of a predetermined number of 7 samples (N-1=7;

N=8) of the signal *Y0* immediately preceding the considered sample of the signal *Y0* (for signal CLK=360Hz), which corresponds to time 22.2222 ms, and dividing this sum by a number N=8 (CLK=360Hz) and squaring such obtained number.

At the same time, by means of the module TH MODULE the value of the threshold TH_PREPROCESSED is systematically decreased every sampling interval of the ECG signal (1/360 s for signal CLK =360Hz) starting from its initial value previously determined by means of the module TH MODULE by multiplying the current threshold value TH_PREPROCESSED, in time instants of receiving successive samples of the ECG signal, by the predetermined and less than one constant value TH_REDUCING_FACTOR = 0.9818 (value for CLK=360Hz).

At the same time, local extremes of the ECG signal are searched for by means of the module EXTREMA MODULE.

After detecting by means of the module EXTREMA MODULE that the considered sample of the ECG signal is the local extreme of the ECG signal, a value of a parameter FACTOR is determined for this sample of the ECG signal by means of the module FACTOR MODULE, which parameter specifies how distinct local extreme the considered sample of the ECG signal is against the neighbouring samples of the ECG signal, and for this purpose the parameter FACTOR is assigned a value the bigger, the greater the amplitude of the considered local extreme represented by the considered sample of the ECG signal than the values of the neighbouring samples of the ECG signal.

Next, after determining the value of the parameter FACTOR for the considered sample of the ECG signal being the local extreme of the ECG signal, the determined value of the parameter FACTOR is compared with the threshold TH_FACTOR previously determined by means of the module TH FACTOR MODULE, and if the value of the parameter FACTOR is greater than the set threshold TH_FACTOR, the value of the parameter FACTOR and the time instant of receiving the considered sample of the ECG signal previously registered by means of the module EXTREMA MODULE, for which time instant the parameter FACTOR has been determined, are stored in a list THRESHOLD_WINDOW_LIST in a memory of the module FACTOR MEMORY, wherein these operations are repeated the required number of times until a time instant of reaching the threshold TH_PREPROCESSED by the signal Y_PREPROCESSED is detected by means of the comparator COMPARATOR, whose value is systematically decreased every sampling interval of the ECG signal (1/360 s) by successive multiplying the value of the threshold TH_PREPROCESSED, in time instants of receiving successive samples of the ECG signal, by the predetermined and less than one constant value TH_REDUCING_FACTOR = 0.9818 (value for 360Hz).

When the time instant of reaching the current value of the threshold TH_PREPROCESSED by the signal Y_PREPROCESSED is detected, a time window SEARCHING_WINDOW is started to be measured by means of the pulse generator SEARCHING WINDOW PULSE GENERATOR for searching for the R-wave in the QRS complex of the ECG signal having a determined minimal length SEARCHING_WINDOW_LEN_MIN = 260ms (which corresponds to duration of 93 samples).

Next, during the time window SEARCHING_WINDOW a maximum value of the signal Y_PREPROCESSED is detected by means of the detection module PEAK DETECTOR and this value is stored in the module PEAK AMPLITUDE MEMORY.

At the same time, during the time window SEARCHING_WINDOW local extremes of the ECG signal are determined by means of the module EXTREMA MODULE, and next, for each one of the samples recognized as a local extreme it is determined, by determining a value of the parameter FACTOR by means of the module FACTOR MODULE, how distinct local extreme the considered sample of the ECG signal is against the neighbouring samples.

For each detected local extreme the value of the parameter FACTOR and a time instant of receiving the considered sample of the ECG signal previously registered by means of the module EXTREMA MODULE, for which time instant the parameter FACTOR has been determined, are stored in a list SEARCHING_WINDOW_LIST in the memory module FACTOR MEMORY.

After a set minimum duration SEARCHING_WINDOW_LEN_MIN = 260ms of the time window SEARCHING_WINDOW the measurement of the time window SEARCHING_WINDOW is terminated by the pulse generator SEARCHING WINDOW PULSE GENERATOR, provided that the maximum value of the signal Y_PREPROCESSED registered during the time window SEARCHING_WINDOW and reduced by the current value of the signal Y_PREPROCESSED reaches a predetermined threshold MIN_DIFF = 0.062 [mV], what is detected by the module PULSE EXTENSION.

After the end of the time window SEARCHING_WINDOW such a value FACTOR_MAX1 of the parameter FACTOR is selected from the list SEARCHING_WINDOW_LIST by means of a module FACTOR SELECTOR, which is the greatest among those stored in the list SEARCHING_WINDOW_LIST and the maximum value FACTOR_MAX1 of the parameter FACTOR considered to be the double amplitude of the R-wave in the QRS complex is stored in a module AMPLITUDE MEMORY, and next, the maximum value FACTOR_MAX1 of the parameter FACTOR is copied to the output memory module OUTPUT MEMORY.

At the same time a time instant of occurrence of the maximum value FACTOR_MAX1 of the parameter FACTOR is stored in the module TIMESTAMP MEMORY, which is also recognized as the time instant of occurrence of the R-wave in the QRS complex, and then the time instant of occurrence of the maximum value FACTOR_MAX1 of the parameter FACTOR is copied to the output memory module OUTPUT MEMORY.

Next, from the list THRESHOLD_WINDOW_LIST in the module FACTOR MEMORY those maximum values of the parameter FACTOR stored in this list are removed by means of the module FACTOR SELECTOR, whose time instant of occurrence precedes by less than a set time period R-TO-R_MIN = 200 ms (which corresponds to 72 samples at signal frequency CLK = 360Hz) since the time instant of occurrence of the R-wave in the QRS complex stored in the output memory module OUTPUT MEMORY.

Next, provided that the number of remaining elements in the list THRESHOLD_WINDOW_LIST is not greater than a set constant MAX_ABOVE_TH = 2, that value FACTOR_MAX2 of the parameter FACTOR is selected from the list THRESHOLD_WINDOW_LIST by means of the module FACTOR SELECTOR, which is the greatest among those stored in the list THRESHOLD_WINDOW_LIST and the maximum value FACTOR_MAX2 of the parameter FACTOR considered to be double the amplitude of the previous R-wave in the QRS complex is stored in the module AMPLITUDE MEMORY, rather than the R-wave previously detected for the value FACTOR_MAX1 of the parameter FACTOR, and then the maximum value FACTOR_MAX2 of the parameter FACTOR is copied to the output memory module OUTPUT MEMORY.

At the same time a time instant of occurrence of the maximum value FACTOR_MAX2 of the parameter FACTOR is stored in the module TIMESTAMP MEMORY, which is also recognized as the time instant of occurrence of the previous R-wave in the QRS complex, rather than the R-wave previously detected for the value FACTOR_MAX1 of the parameter FACTOR, and then the time instant of occurrence of the maximum value FACTOR_MAX2 of the parameter FACTOR is copied to the output memory module OUTPUT MEMORY.

Next, the sum value of the values of the parameters FACTOR_MAX1 obtained so far is updated by means of the module TH FACTOR MODULE by adding the current value FACTOR_MAX1 to the previous sum value and a counter of performed algorithm work cycles is updated and on this basis an arithmetic mean of the values FACTOR found so far within time windows is calculated, and then the parameter TH_FACTOR is assigned that value among the pair MIN_TH_FACTOR = 1.22 [mV] and NEW_TH_FACTOR, which is not less than the second value in the pair MIN_TH_FACTOR and NEW_TH_FACTOR, wherein NEW_TH_FACTOR is the product of a parameter EXPECTED_TH_FACTOR = 0.48 and of a weighted mean with weights, respectively, (1 - TH_FACTOR_LAST_PEAK_COEF) = 0.6 and TH_FACTOR_LAST_PEAK_COEF = 0.4 of the arithmetic mean of values of the parameter FACTOR for the R-waves in the QRS complex found so far and the value of the parameter FACTOR_MAX1 for the last found R-wave.

Next, the sum value of the maximum values of the signal Y_PREPROCESSED obtained so far is updated by means of the module TH MODULE by adding the current maximum value Y_PREPROCESSED to the previous sum value and the counter of performed algorithm work cycles is updated and on this basis an arithmetic mean of the values Y_PREPROCESSED found so far within time windows is calculated, and next, the parameter TH_PREPROCESSED is assigned a new value, which is a weighted mean with weights, respectively, (1 - TH_PREPROCESSED_LAST_PEAK_COEF) = 0.3 and TH_PREPROCESSED_LAST_PEAK_COEF = 0.7 of the arithmetic mean of maximum values of the signal Y_PREPROCESSED found so far within time windows for detecting R-waves in the QRS complex and of the maximum value of the signal Y_PREPROCESSED for the current time window SEARCHING_WINDOW, in which the last R-wave has been identified.

Next, by means of the module REFRACTORY WINDOW PULSE GENERATOR a measurement of a time window REFRACTORY_WINDOW is started, the end of which occurs after time REFRACTORY_LEN = 200ms since the time instant of detecting the last R-wave.

Next, during REFRACTORY_WINDOW all the stored elements on the lists THRESHOLD_WINDOW_LIST and SEARCHING_WINDOW_LIST are removed from the module FACTOR MEMORY in order to prepare the module for the next work cycle.

After terminating the measurement of the time window REFRACTORY_WINDOW, monitoring of the signal Y_PREPROCESSED generated at the output of the module PREPROCESSING MODULE by means of the comparator COMPARATOR is resumed, and then the cycle is repeated the desired number of times.

In an advantageous embodiment of the invention the local extremes of the ECG signal are searched for by means of the module EXTREMA MODULE in such a way that

- after receiving the considered sample of the ECG signal by means of the measuring module ECG MODULE and storing this sample of the ECG signal by means of the module BUF, a maximum value LOCAL_EXT_MAX_LEFT and a minimum value LOCAL_EXT_MIN_LEFT are determined by means of the EXTREMA MODULE for a set of predetermined number LOCAL_EXT_LEN = 6 samples (which corresponds to time 0.017 ms at signal frequency CLK = 360 Hz) of the ECG signal stored in the buffer BUF that immediately precede the considered sample and of the considered sample of the ECG signal, and then the maximum value LOCAL_EXT_MAX_LEFT and the minimum value LOCAL_EXT_MIN_LEFT are stored by means of the module EXTREMA MODULE, and also

- after receiving by means of the measuring module ECG_MODULE and collecting in the buffer BUF the predetermined number LOCAL_EXT_LEN = 6 samples of the ECG signal that follow immediately after the considered sample of the ECG signal, a maximum value LOCAL_EXT_MAX_RIGHT and a minimum value LOCAL_EXT_MIN_RIGHT are determined by means of the module EXTREMA MODULE for a set of predetermined number LOCAL_EXT_LEN of samples of the ECG signal and of the considered sample of the ECG signal, and then these maximum value LOCAL_EXT_MAX_RIGHT and minimum value LOCAL_EXT_MIN_RIGHT are stored by means of the module EXTREMA MODULE.

Next, the previously determined maximum value LOCAL_EXT_MAX_LEFT and the previously determined minimum value LOCAL_EXT_MIN_LEFT are successively compared by means of the module EXTREMA MODULE, for the predetermined number LOCAL_EXT_LEN of samples of the ECG signal immediately preceding the considered sample of the ECG signal and stored in the buffer BUF, with the value Y of the considered sample of the ECG signal, and also the previously determined maximum value LOCAL_EXT_MAX_ RIGHT and the previously determined LOCAL_EXT_MIN_RIGHT are successively compared by means of the module EXTREMA MODULE, for the predetermined number LOCAL_EXT_LEN = 6 samples of the ECG signal that follow immediately after the considered sample of the ECG signal, with the value Y of the considered sample of the ECG signal. If the result of the comparisons shows equality with the value Y of the considered sample of the ECG signal for at least one of the maximum values LOCAL_EXT_MAX_LEFT and LOCAL_EXT_MAX_RIGHT or the minimum values LOCAL_EXT_MIN_LEFT and LOCAL_EXT_MIN_RIGHT obtained previously for the groups of samples immediately preceding and immediately following the considered sample of the ECG signal, then the considered sample of the ECG signal is recognized as a local extreme.

In another advantageous embodiment of the invention, the value of the parameter FACTOR, which specifies how distinct local extreme the considered sample of the ECG signal is against the neighbouring samples of the ECG signal, is determined by means of the module FACTOR MODULE in such a way that

a minimum value MIN_LEFT and a maximum value MAX_LEFT of samples of the ECG signal are determined for a predetermined number MAX_WIDTH = 17 samples (which corresponds to 0,047ms for signal CLK=360Hz) of the ECG signal that immediately precede the considered sample and are stored in the buffer BUF, and then, this maximum value MAX_LEFT and this minimum value MIN_LEFT are stored by means of the module FACTOR MODULE for the predetermined number MAX_WIDTH = 17 samples of the ECG signal that immediately precede the considered sample, and also

- after receiving by means of the measuring module ECG MODULE and collecting in the buffer BUF the predetermined number MAX_WIDTH = 17 samples (which corresponds to 0,047ms for signal CLK=360Hz) of the ECG signal that follow immediately after the considered sample of the ECG signal, a maximum value MAX_RIGHT and a minimum value MIN_RIGHT are determined by means of the module FACTOR MODULE for the predetermined number MAX_WIDTH of samples of the ECG signal, and then the maximum value MAX_RIGHT and the minimum value MIN_RIGHT are stored by means of the module FACTOR MODULE for the predetermined number MAX_WIDTH = 17 samples of the ECG signal that follow immediately after the considered sample of signal, and the value of the parameter FACTOR is determined by taking as the parameter FACTOR the value of that one among the pair of the parameters FACTOR_MAX and FACTOR_MIN, which is not less than the other one, where the values of the parameters FACTOR_MAX and FACTOR_MIN are determined according to the formulas:

$$FACTOR\_MAX = (Y - MIN\_LEFT) + (Y - MIN\_RIGHT)$$

$$FACTOR\_MIN = (MAX\_LEFT - Y) + (MAX\_RIGHT - Y)$$

wherein Y is the value of the considered sample of the ECG signal, for which the factor FACTOR is determined.

**Claims**

1.  A device for automatic QRS complex detection in an electrocardiogram signal having connection with a measurement module ECG MODULE of the ECG signal, wherein the device comprises:

    - a module BUF for buffering data supplied to its input connected to an output of the module ECG MODULE,
    - a module PREPROCESSING MODULE, whose first input is connected to an output of the module BUF, generating at its output a signal Y_PREPROCESSED obtained by generating a signal *Y0* being the difference between a value *Y* of the considered sample of the ECG signal and a sample value of the ECG signal previously stored in the buffer BUF, which has been received a predetermined number *Nd* of samples earlier, and determining a signal *Y1* being the sum of the considered sample of the signal *Y0* and a value of a predetermined number N-1 of samples of the signal *Y0* immediately preceding the considered sample of the signal *Y0,* and dividing this sum by a number N and squaring such obtained number,

- a module TH MODULE for setting an initial value and determining a current threshold value TH_PREPROCESSED;

- a comparator COMPARATOR, whose first input is connected to the output of the module PREPROCESSING MODULE, and whose second input is connected to an output of the TH MODULE;

- a module SEARCHING WINDOW PULSE GENERATOR generating a time window SEARCHING_WINDOW for searching for the R-wave in the QRS complex of the ECG signal, whose first input is connected to an output of the comparator COMPARATOR, and whose output is connected to a second input of the module PREPROCESSING MODULE,

- a module PEAK DETECTOR for detecting a maximum value of the signal Y_PREPROCESSED, whose first input is connected to the output of the module PREPROCESSING MODULE and whose second input is connected to the output of the module SEARCHING WINDOW PULSE GENERATOR,

- a module PEAK AMPLITUDE MEMORY for storing a maximum value of the signal Y_PREPROCESSED, whose input is connected to a first output of the module PEAK DETECTOR and whose output is connected to an input of the module TH MODULE,

- memory modules AMPLITUDE MEMORY and TIMESTAMP MEMORY for storing, respectively, an amplitude value and occurrence time of a parameter corresponding to the detected R-wave, as well as a memory module OUTPUT MEMORY for storing values from the memories AMPLITUDE MEMORY and TIMESTAMP MEMORY, whose outputs are connected to inputs of the memory module OUTPUT MEMORY,

**characterized in that** the said device furthermore comprises:

- a module PULSE EXTENSION for detecting that the maximum value of the signal Y_PREPROCESSED reduced by the current value of the signal Y_PREPROCESSED reaches a predetermined threshold MIN_DIFF, whose first input is connected to the output of the module PREPROCESSING MODULE, whose second input is connected the output of the module PEAK AMPLITUDE MEMORY and whose output is connected to a second input of the module SEARCHING WINDOW PULSE GENERATOR,

- a module REFRACTORY WINDOW PULSE GENERATOR for measuring a time window REFRACTORY_WINDOW, the end of which occurs after time REFRACTORY_LEN since a time instant of detecting the last R-wave, whose input is connected to a second output of the module PEAK DETECTOR and whose output is connected to a third input of the module PREPROCESSING MODULE,

- a module EXTREMA MODULE for searching for local extremes of the ECG signal, whose first input is connected to the output of the module BUF, whose second input is connected to the output of the module REFRACTORY WINDOW PULSE GENERATOR, and whose third input is connected to the second output of the module SEARCHING WINDOW PULSE GENERATOR,

- a module FACTOR MODULE for determining a value of a parameter FACTOR, which specifies how distinct local extreme the considered sample of the ECG signal is against the neighbouring samples of the ECG signal, whose input is connected to an output of the module EXTREMA MODULE,

- a module TH FACTOR MODULE for determining and updating a threshold TH_FACTOR, whose input is connected to the output of the module AMPLITUDE MEMORY,

- a memory module FACTOR MEMORY for storing a list THRESHOLD_WINDOW_LIST comprising values of the parameter FACTOR greater than the set threshold TH_FACTOR and time instants of receiving associated samples of the ECG signal, for which the parameter FACTOR has been determined, whose first input is connected to an output of the module FACTOR MODULE, whose second input is connected to the output of the module REFRACTORY WINDOW PULSE GENERATOR, whose third input is connected to the output of the module SEARCHING WINDOW PULSE GENERATOR, and whose fourth input is connected to an output of the module TH FACTOR MODULE,

- a module FACTOR SELECTOR for selecting the greatest one among the values stored in the list SEARCHING_WINDOW_LIST, whose first input is connected to an output of the module FACTOR MEMORY, whose second input is connected to the output of the module SEARCHING WINDOW PULSE GENERATOR, and whose two outputs are connected, respectively, to the module TIMESTAMP MEMORY and to the module AMPLITUDE MEMORY.

**Patentansprüche**

1. Eine Vorrichtung zur automatischen Erkennung von QRS-Komplexen in einem Elektrokardiogrammsignal mit Verbindung mit einem Messmodul ECG MODULE des EKG-Signals, wobei die Vorrichtung umfasst:

   - ein Modul BUF zum Puffern von Daten, die an seinen Eingang geliefert werden, der mit einem Ausgang des Moduls ECG MODULE verbunden ist,
   - ein Modul PREPROCESSING MODULE, dessen erster Eingang mit einem Ausgang des Moduls BUF verbunden ist, das an seinem Ausgang ein Signal Y_PREPROCESSED erzeugt, das durch Erzeugen eines Signals $Y0$ erhalten wird, das die Differenz zwischen einem Wert $Y$ der betrachteten Probe des EKG-Signals und einem zuvor im Puffer BUF gespeicherten Probenwert des EKG-Signals ist, der eine vorbestimmte Anzahl $Nd$ Proben früher empfangen wurde, und durch Bestimmen eines Signals $Y1$, das die Summe der betrachteten Probe des Signals $Y0$ und eines Werts einer vorbestimmten Anzahl $N-1$ Proben des Signals $Y0$ ist, die der betrachteten Probe des Signals $Y0$ unmittelbar vorausgehen, und durch Teilen dieser Summe durch eine Zahl $N$ und Quadrieren dieser erhaltenen Zahl,
   - ein Modul TH MODULE zum Einstellen eines Anfangswerts und Bestimmen eines aktuellen Schwellenwerts TH_PREPROCESSED;
   - ein Komparator COMPARATOR, dessen erster Eingang mit dem Ausgang des Moduls PREPROCESSING MODULE und dessen zweiter Eingang mit einem Ausgang des TH MODULE verbunden ist;
   - ein Modul SEARCHING WINDOW PULSE GENERATOR, das ein Zeitfenster SEARCHING_WINDOW zum Suchen der R-Welle im QRS-Komplex des EKG-Signals erzeugt, dessen erster Eingang mit einem Ausgang des Komparators COMPARATOR verbunden ist und dessen Ausgang mit einem zweiten Eingang des Moduls PREPROCESSING MODULE verbunden ist,
   - ein Modul PEAK DETECTOR zum Erkennen eines Maximalwerts des Signals Y_PREPROCESSED, dessen erster Eingang mit dem Ausgang des Moduls PREPROCESSING MODULE verbunden ist und dessen zweiter Eingang mit dem Ausgang des Moduls SEARCHING WINDOW PULSE GENERATOR verbunden ist,
   - ein Modul PEAK AMPLITUDE MEMORY zum Speichern eines Maximalwerts des Signals Y_PREPROCESSED, dessen Eingang mit einem ersten Ausgang des Moduls PEAK DETECTOR verbunden ist und dessen Ausgang mit einem Eingang des Moduls TH MODULE verbunden ist,
   - Speichermodule AMPLITUDE MEMORY und TIMESTAMP MEMORY zum Speichern eines Amplitudenwert und Auftretenszeit eines Parameters, der der erkannten R-Welle entspricht, sowie ein Speichermodul OUTPUT MEMORY zum Speichern von Werten aus den Speichern AMPLITUDE MEMORY und TIMESTAMP MEMORY, dessen Ausgänge mit Eingängen des Speichermoduls OUTPUT MEMORY verbunden sind,

   **dadurch gekennzeichnet, dass**
   die Vorrichtung außerdem umfasst:

   - ein Modul PULSE EXTENSION zum Erkennen, dass der Maximalwert des Signals Y_PREPROCESSED, reduziert um den aktuellen Wert des Signals Y_PREPROCESSED, einen vorgegebenen Schwellenwert MIN_DIFF erreicht, dessen erster Eingang mit dem Ausgang des Moduls PREPROCESSING MODULE verbunden ist, dessen zweiter Eingang mit dem Ausgang des Moduls PEAK AMPLITUDE MEMORY verbunden ist und dessen Ausgang mit einem zweiten Eingang des Moduls SEARCHING WINDOW PULSE GENERATOR verbunden ist,
   - ein Modul REFRACTORY WINDOW PULSE GENERATOR zum Messen eines Zeitfensters REFRACTORY_WINDOW, dessen Ende nach der Zeit REFRACTORY_LEN seit einem Zeitpunkt der Erkennung der letzten R-Welle eintritt, dessen Eingang mit einem zweiten Ausgang des Moduls PEAK DETECTOR verbunden ist und dessen Ausgang mit einem dritten Eingang des Moduls PREPROCESSING MODULE verbunden ist,
   - ein Modul EXTREMA MODULE zum Suchen nach lokalen Extrema des EKG-Signals, dessen erster Eingang ist mit dem Ausgang des Moduls BUF verbunden ist, dessen zweiter Eingang mit dem Ausgang des Moduls REFRACTORY WINDOW PULSE GENERATOR verbunden ist und dessen dritter Eingang mit dem zweiten Ausgang des Moduls SEARCHING WINDOW PULSE GENERATOR verbunden ist,
   - ein Modul FACTOR MODULE zum Bestimmen eines Werts eines Parameters FACTOR, der angibt, wie deutlich sich das lokale Extrem der betrachteten Probe des EKG-Signals von den benachbarten Proben des EKG-Signals unterscheidet, dessen Eingang mit einem Ausgang des Moduls EXTREMA MODULE verbunden ist,
   - ein Modul TH FACTOR MODULE zum Bestimmen und Aktualisieren eines Schwellenwerts TH_FACTOR, dessen Eingang mit dem Ausgang des Moduls AMPLITUDE MEMORY verbunden ist,
   - ein Speichermodul FACTOR MEMORY zum Speichern einer Liste THRESHOLD_WINDOWL_LIST, die Werte des Parameters FACTOR umfasst, die größer als der festgelegte Schwellenwert TH_FACTOR sind, und Zeitpunkte des Empfangs zugehöriger Proben des EKG-Signals, für die der Parameter FACTOR bestimmt wurde, dessen erster Eingang ist mit einem Ausgang des Moduls FACTOR MODULE verbunden, dessen zweiter Eingang mit dem Ausgang des Moduls REFRACTORY WINDOW PULSE GENERATOR verbunden ist, dessen dritter Eingang mit dem Ausgang des Moduls SEARCHING WINDOW PULSE GENERATOR verbunden ist und dessen vierter Eingang mit einem Ausgang des Moduls TH FACTOR MODULE verbunden ist,
- ein Modul FACTOR SELECTOR zum Auswählen des größten Wertes unter den in der Liste SEARCHING_WINDOWL_LIST gespeicherten Werten, dessen erster Eingang mit einem Ausgang des Moduls FACTOR MEMORY verbunden ist, dessen zweiter Eingang mit dem Ausgang des Moduls SEARCHING WINDOW PULSE GENERATOR verbunden ist und dessen zwei Ausgänge jeweils mit dem Modul TIMESTAMP MEMORY und dem Modul AMPLITUDE MEMORY verbunden sind.

## Revendications

1. Dispositif de détection automatique de complexe QRS dans un signal d'électrocardiogramme ayant une connexion avec un module de mesure ECG MODULE du signal ECG, dans lequel le dispositif comprend :

   - un module BUF de mise en mémoire tampon des données fournies à son entrée connectée à une sortie du module ECG MODULE,
   - un module PREPROCESSING MODULE, dont la première entrée est connectée à une sortie du module BUF, générant à sa sortie un signal Y_PREPROCESSED obtenu en générant un signal $Y0$ étant la différence entre une valeur Y de l'échantillon considéré du signal ECG et une valeur d'échantillon du signal ECG précédemment stockée dans la mémoire tampon BUF, qui a été reçue un nombre prédéterminé Nd d'échantillons plus tôt, et en déterminant un signal $Y1$ étant la somme de l'échantillon considéré du signal $Y0$ et d'une valeur d'un nombre prédéterminé N-1 d'échantillons du signal $Y0$ précédant immédiatement l'échantillon considéré du signal $Y0,$ et en divisant cette somme par un nombre N et en élevant au carré ce nombre obtenu,
   - un module TH MODULE pour fixer une valeur initiale et déterminer une valeur de seuil actuelle TH_PREPROCESSED;
   - un comparateur COMPARATOR, dont la première entrée est connectée à la sortie du module PREPROCESSING MODULE, et dont la deuxième entrée est connectée à une sortie du TH MODULE;
   - un module SEARCHING WINDOW PULSE GENERATOR générant une fenêtre temporelle SEARCHING_WINDOW pour la recherche de l'onde R dans le complexe QRS du signal ECG, dont la première entrée est connectée à une sortie du comparateur COMPARATOR, et dont la sortie est connectée à une seconde entrée du module PREPROCESSING MODULE,
   - un module PEAK DETECTOR pour la détection d'une valeur maximale du signal Y_PREPROCESSED, dont la première entrée est connectée à la sortie du module PREPROCESSING MODULE et dont la seconde entrée est connectée à la sortie du module SEARCHING WINDOW PULSE GENERATOR,
   - un module PEAK AMPLITUDE MEMORY pour le stockage d'une valeur maximale du signal Y_PREPROCESSED, dont l'entrée est connectée à une première sortie du module PEAK DETECTOR et dont la sortie est connectée à une entrée du module TH MODULE,
   - des modules mémoire AMPLITUDE MEMORY et TIMESTAMP MEMORY pour le stockage, respectivement, d'une valeur d'amplitude et d'un temps d'occurrence d'un paramètre correspondant à l'onde R détectée, ainsi qu'un module mémoire OUTPUT MEMORY pour stocker des valeurs des mémoires AMPLITUDE MEMORY et TIMESTAMP MEMORY, dont les sorties sont connectées aux entrées du module mémoire OUTPUT MEMORY,

   **caractérisé en ce que** ledit dispositif comprend en outre :

   - un module PULSE EXTENSION pour détecter que la valeur maximale du signal Y_PREPROCESSED diminuée de la valeur courante du signal Y_PREPROCESSED atteint un seuil prédéterminé MIN_DIFF, dont la première entrée est connectée à la sortie du module PREPROCESSING MODULE, dont la seconde entrée est connectée à la sortie du module PEAK AMPLITUDE MEMORY et dont la sortie est connectée à une seconde entrée du module SEARCHING WINDOW PULSE GENERATOR,
   - un module REFRACTORY WINDOW PULSE GENERATOR pour mesurer une fenêtre temporelle REFRACTORY_WINDOW dont la fin intervient après le temps REFRACTORY_LEN depuis un instant de détection de la dernière onde R, dont l'entrée est connectée à une seconde sortie du module PEAK DETECTOR et dont la sortie est connectée à une troisième entrée du module PREPROCESSING MODULE,
   - un module EXTREMA MODULE pour rechercher des extrêmes locaux du signal ECG, dont première entrée est connectée à la sortie du module BUF, dont la deuxième entrée est connectée à la sortie du module REFRACTORY WINDOW PULSE GENERATOR, et dont la troisième entrée est connectée à la deuxième sortie du module SEARCHING WINDOW PULSE GENERATOR,

- un module FACTOR MODULE pour déterminer une valeur d'un paramètre FACTOR, qui spécifie à quel point l'extrême local de l'échantillon considéré du signal ECG est distinct des échantillons voisins du signal ECG, dont l'entrée est connectée à une sortie du module EXTREMA MODULE,

- un module TH FACTOR MODULE pour déterminer et mise à jour un seuil TH_FACTOR, dont l'entrée est connectée à la sortie du module AMPLITUDE MEMORY,

- un module mémoire FACTOR MEMORY pour stocker une liste THRESHOLD_WINDOW_LIST comprenant des valeurs du paramètre FACTOR supérieures au seuil TH_FACTOR fixé et des instants de temps de réception d'échantillons associés du signal ECG, pour lesquels le paramètre FACTOR a été déterminé, dont la première entrée est connectée à une sortie du module FACTOR MODULE, dont une deuxième entrée est connectée à la sortie du module REFRACTORY WINDOW PULSE GENERATOR, dont la troisième entrée est connectée à la sortie du module SEARCHING WINDOW PULSE GENERATOR, et dont la quatrième entrée est connectée à une sortie du module TH FACTOR MODULE,

- un module FACTOR SELECTOR pour sélectionner la plus grande parmi les valeurs stockées dans la liste SEARCHING_WINDOW_LIST, dont la première entrée est connectée à une sortie du module FACTOR MEMORY, dont la deuxième entrée est connectée à la sortie du module SEARCHING WINDOW PULSE GENERATOR, et dont les deux sorties sont connectées, respectivement, au module TIMESTAMP MEMORY et au module AMPLITUDE MEMORY.

**Fig. 1**

**Fig. 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GUTIÉRREZ-RIVAS, RAQUEL et al.** Novel real-time low-complexity QRS complex detector based on adaptive thresholding. *IEEE Sensors Journal*, 2015, vol. 15 (10), 6036-6043